# EUROPEAN PATENT SPECIFICATION

(11) **EP 2 184 052 B1**
(45) Date of publication and mention of the grant of the patent: **20.09.2017**
(21) Application number: 08019657.9
(22) Date of filing: 11.11.2008
(51) Int. Cl.: A61K 8/92, A61Q 5/00, A61Q 17/04

(54) **Composition for hair**
Haarzusammensetzung
Composition pour cheveux

(43) Date of publication of application: 12.05.2010
(73) Proprietor: Kao Germany GmbH, 64297 Darmstadt (DE)
(72) Inventor: Hoffmann, Martin, 64673 Zwingenberg (DE); Ast, Andrea, 64283 Darmstadt (DE); Arbter, Claudia, 56235 Ransbach-Baumbach (DE)
(74) Representative: Grit, Mustafa

(56) References cited:
- EP-A- 1 166 760
- EP-A1- 2 179 986
- WO-A-2004/110366
- WO-A-2005/084619
- WO-A-2005/097059
- WO-A-2006/010214
- WO-A1-2005/041914
- WO-A1-2008/136889
- WO-A2-2009/138978
- US-A- 5 843 193
- US-A- 6 146 616
- US-A1- 2005 276 761
- US-A1- 2008 014 153
- DATABASE GNPD [Online] MINTEL February 2008 'Shampoo' Database accession no. 862723
- DATABASE GNPD [Online] MINTEL February 2008 'Conditioner' Database accession no. 862790
- DATABASE GNPD [Online] MINTEL November 2007 'Moisturising colour care conditioner' Database accession no. 822393
- DATABASE GNPD [Online] MINTEL April 2004 'Quench nourishing conditioner' Database accession no. 10168118
- DATABASE GNPD [Online] MINTEL September 2004 'Intense moisturising shampoo' Database accession no. 10189184
- DATABASE GNPD [Online] MINTEL September 2004 'Styling gel' Database accession no. 10186519
- DATABASE GNPD [Online] MINTEL June 2008 'Protect & shine serum' Database accession no. 925793
- DATABASE GNPD [Online] MINTEL October 2003 'Contour hair creme' Database accession no. 231490
- DATABASE GNPD [Online] MINTEL August 2008 'Daily shampoo' Database accession no. 963731
- DATABASE GNPD [Online] MINTEL June 2008 'Conditioner' Database accession no. 932891
- DATABASE GNPD [Online] MINTEL April 2004 'Papaya moisture care shampoo' Database accession no. 10167359
- DATABASE GNPD [Online] MINTEL April 2004 'Papaya moisture care conditioner' Database accession no. 10167372
- DATABASE GNPD [Online] MINTEL March 2008 'Fortifying shampoo' Database accession no. 873148
- DATABASE GNPD [Online] MINTEL August 2004 'Defrizz & smoothing straight balm' Database accession no. 10180618
- DATABASE GNPD [Online] MINTEL 'Volume and body spray' Database accession no. 10180621
- DATABASE GNPD [Online] MINTEL November 2006 'Conditioner' Database accession no. 617809
- DATABASE GNPD [Online] MINTEL October 2007 'Peppermint and Primrose Daily Shampoo' Database accession no. 779157
- DATABASE GNPD [Online] MINTEL February 2007 'Hair Masque' Database accession no. 654815
- DATABASE GNPD [Online] MINTEL February 2007 'Conditioner' Database accession no. 654661
- DATABASE GNPD [Online] MINTEL March 2008 'Conditioner' Database accession no. 882574
- DATABASE GNPD [Online] MINTEL March 2007 'Decadent Soft Movement C-Gel' Database accession no. 681973

## Description

Present invention relates to a composition for hair comprising at least one natural oil and at least one UV filter. Compositions of the present invention effectively protect hair from UV rays and daily sunlight exposure and therefore prevent hair damage and hair colour fading.

Natural oils have traditionally been used in personal care products. Hair conditioning compositions comprising natural oils have been known and presented in textbooks well known by the skilled in the cosmetic art.

UV filters have also been used in cosmetic compositions for hair either for protection of the ingredients from oxidation and/or colour fading of the cosmetic composition and/or protecting hair and also skin from damaging UV rays which causes at the same time colour fading.

Inventors of the present invention have surprisingly found out that a composition comprising Passiflora oil (Passiflora edulis, Passiflora incarnate) and Polysilicone-15 is protecting hair from damaging UV rays especially effectively which is observed as synergistic protection of hair colour exposed heavily to UV rays. Accordingly, the first object of the present invention is a composition for hair comprising Polysilicone-15 and Passiflora oil (Passiflora edulis, Passiflora incarnate).

Further object of the present invention is the use of the composition comprising Polysilicone-15 and Passiflora oil (Passiflora edulis, Passiflora incarnate)for protecting hair from UV rays.

The compositions of the present invention condition hair optimally in terms of combing, shine, elasticity, manageability and also do not reduce hair volume and body. Therefore, another object is the use of the compositions of the present invention for conditioning hair.

Still another object of present invention is the use of the composition of the present invention for cleansing and conditioning hair.

Still further object of the present invention is the use of the composition of the present invention for conditioning hair after cleansing it with a cleansing composition.

According to present invention, in a broader sense, compositions can be either cleansing composition based on at least one cleansing surfactant and comprising Polysilicone-15 and Passiflora oil (Passiflora edulis, Passiflora incarnate) or a composition applied after cleansing hair and comprising the two ingredients. It has been observed that the effects are observed stronger when both compositions cleansing and composition applied after cleansing hair comprises the both ingredients. Therefore, further object of the present invention is a process for conditioning hair wherein a cleansing composition comprising Polysilicone-15 and Passiflora oil (Passiflora edulis, Passiflora incarnate) is applied onto hair and rinsed off from hair and a second composition without a cleansing effect comprising Polysilicone-15 and Passiflora oil (Passiflora edulis, Passiflora incarnate) is applied onto hair and optionally rinsed off from hair after certain processing time.

Compositions of the present invention are preferably aqueous and comprise water. Water concentration may depend on the application form but typically and generally it is more than 50% in case of cleansing compositions and also rinse-off, as well as leave in compositions. It is certainly within the meaning of the present invention that an anhydrous composition will provide the same benefits mentioned above when applied as a leave in composition.

The amount of Polysilicone-15 ranges typically from 0.01 % to 7.5%, more preferably from 0.05 % to 5 % by weight, calculated to the total composition. Concentration of Passiflora oil (Passiflora edulis, Passiflora incarnate) is in the range of 0.01 to 5%, preferably 0.05 to 4%, more preferably 0.1 to 2.5% and most preferably 0.1 to 1.5% by weight calculated to total composition.

In one of the preferred embodiment of the present invention, compositions additionally comprise at least one polyol. The term polyol refers to a compound carrying 2 or more hydroxyl groups in its molecule.

Non-limiting suitable examples to the polyols preferred within the scope of the present invention are ethylene glycol and its homo polymers with varying molecular weight known with their INCI names such as diethyleneglycol, triethylene glycol and PEG with 4 to 800 ethylene glycol units, propylene glycol and its homo polymers with varying molecular weight known with their INCI names such as dipropylene glycol, tripropylene glycol and PPG with 4 to 50 propylene glycol units and glycerin and its homo polymers known with their INCI names diglycerin, triglycerin and polyglycerin with 4 to 40 glycerin units. Any of the homopolymers mentioned here can be liquid, semisolid, or solid. In the preferred from of the invention suitable polyols are the ones either liquid or semiliquid at ambient temperature. The most preferred are liquid ones at ambient temperature such as propylenegylcol, dipropylene glycol, PPG-9, diethylene glycol, triethylene glycol and glycerin.

Concentration of one or more polyol in the composition is varying between 0.01 and 25%, preferably 0.1 and 20%, more preferably 0.25 and 15% and most preferably 0.25 and 10% by weight calculated to total composition. The concentration ranges mentioned here refer to the total concentration of polyols in case there are more than one polyol present in the compositions.

Compositions of the present invention can be either a conditioning -cleansing composition or a conditioning composition typically used after using cleansing compositions

Composition of the present invention comprises hair-conditioning agents in any type of composition. Conditioning agents can be selected from oily substances, non-ionic substances, cationic amphiphilic ingredients, cationic polymers or their mixtures.

Oily substances are selected from such as volatile or non-volatile silicone oils, natural oils other than falling in the definition of claim 1 and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule, natural oils such as olive oil, almond oil, avocado oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyethyleneglycol mono or di fatty acid esters having general formula

R₂ CO (O CH₂ CH₂)ₙ OH

or

R₂ CO (O CH₂ CH₂)ₙ O OC R₃

wherein R₂ and R₃ are independent from each other saturated, unsaturated or branched or non-branched alkyl chain with 7 to 21 C atoms and n is typically 2 - 100.

In another preferred from of the present invention, compositions comprise at least one cationic conditioning agent. Cationic conditioning agents are cationic polymers and cationic surfactants. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose and its derivatives, cationic guar gum and its derivatives, cationic Caesalpinia spinosa gum and its derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Cationic surfactants suitable for the present invention are according to the general formula 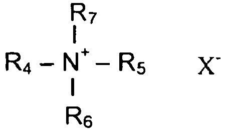 where R₄ is a saturated or unsaturated, branched or straight alkyl chain with 8-22 C atoms or

R₈ CO NH (CH₂)ₙ

where R₈ is saturated or unsaturated, branched or straight alkyl chain with 7-21 C atoms and n has value of 1 - 4,
or

R₉ CO O (CH₂)ₙ

where R₉ is saturated or unsaturated, branched or straight alkyl chain with 7-21 C atoms and n has value of 1 - 4, and
R₅ is hydrogen or unsaturated or saturated, branched or straight alkyl chain with 1 - 22 C atoms or

R₈ CO NH (CH₂)ₙ

or

R₉ CO O (CH₂)ₙ

where R₈, R₉ and n are same as above.

R₆ and R₇ are hydrogen or lower alkyl chain with 1 to 4 carbon atoms, and X is anion such as chloride, bromide, methosulfate.

Typical examples of those ingredients are cetyltrimethyl ammonium chloride, steartrimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropylamine known with a trade name Lexamine S13 from Inolex and behenamidopropyl dimethyl amine available under the tade name Amidet APA 22 from Kao Chemicals.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicon oil and derivatives and cationic surfactants is in the range of 0.01 - 10% by weight, preferably 0.01 - 7.5% by weight, more preferably 0.05 - 5% and most preferably 0.1 - 5% by weight calculated to the total composition. It should be noted that especially non-cleansing conditioning type of the products contain higher concentrations (of the above mentioned concentrations) of the cationic surfactants which at the same time, if desired, can be emulsifying agent. In cleansing and conditioning type of preparations, concentration of cationic surfactants is towards the lower end of the above mentioned concentration, approximately below 1% by weight.

Compositions of the present invention are cleansing compositions (cleansing-conditioning composition). Cleansing compositions of the present invention comprise at least one surfactant selected from anionic, non-ionic and/or amphoteric or zwitterionic surfactants at a concentration range of 1 to 50%, preferably 5 to 40% and more preferably 5 to 30%, and most preferably 5 to 25% by weight, calculated to the total composition.

In one of the preferred embodiment of the present invention cleansing composition of the present invention, comprises at least one anionic, at least one nonionic surfactant. More preferably, the compositions further comprise additionally at least one amphoteric surfactant.

Anionic surfactants suitable within the scope of the invention are preferably present in an amount from 1 to about 30%, preferably 2 to 20% and most preferably 2 - 15%, by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁₀-(C₂H₄O)ₙ-O-CH₂COOX,

wherein R₁₀ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 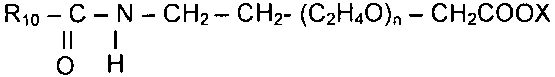 wherein R₁₀ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.
It is also possible to use mixtures of several anionic surfactants, for example an ether sulfate and a polyether carboxylic acid or alkyl amidoether carboxylic acid.

Further suitable anionic surfactants are also C₈-C₂₂-acyl aminocarboxylic acids or the water-soluble salts thereof. Especially preferred are N-lauroyl glutamate and Cocoyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine, N-lauroyl aminopropyl glycine, cocoyl taurate preferably in form of the water-soluble alkali or ammonium, in particular the sodium salts thereof, preferably in admixture with the above-named anionic surfactants.

Further surfactants in the shampoo compositions according to the invention are nonionic surfactants especially in admixture with anionic surfactants.

Especially suited are alkyl polyglucosides of the general formula

R₁₁-O-(R₁₂O)ₙ-Zₓ,

wherein R₁₁ is an alkyl group with 8 to 18 carbon atoms, R₁₂ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

Total concentration of nonionic surfactants in compositions of the present invention is in the range of 1 to 20%, preferably 1 to 15% and more preferably 1 to 10% by weight calculated to total composition.

As further surfactant component, in another preferred form of invention, the compositions especially those of cleansing and conditioning according to the invention comprise at least one amphoteric or zwitterionic surfactant in an amount from 0.5 % to 15 %, preferably from 1 % to 10 %, more preferably from 1 % to 7.5 %by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio is preferably be in the range of 10:1 to 1:1, more preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 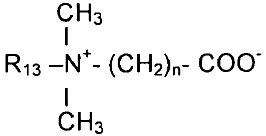 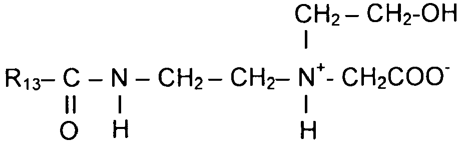 wherein R₁₃ is a C₈-C₁₈-alkyl group and n is 1 to 3;
sulfobetaines of the structure 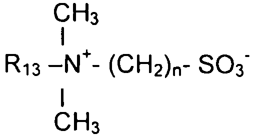 wherein R₁₃ and n are same as above;
and amidoalkyl betaines of the structure 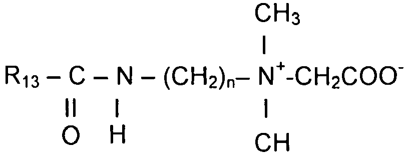 wherein R₁₃ and n are same as above.

In another preferred form of the invention, cleansing composition of the present invention comprise at least one anionic, at least one non-ionic and at least one amphoteric surfactants.

Solubilizers may be added to the compositions, in particular cleansing compositions, especially when oily substances are chosen as conditioning agents and fragrance oils with highly lipophilic properties. Typical solubilizers may be hydrogenated castor oil known with the trade mark Cremophor RH series from BASF. It should be noted that the surfactant mixture as well be a good solubilizer for fragrance oils. Typical concentration of the solubilizers can be in the range of 0.01 - 2% by weight, preferably 0.1 - 1% by weight, calculated to total composition.

Further conditioning additives are hair conditioning and/or styling polymers into either cleansing or conditioning type compositions. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning composition of any type of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl - methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl - aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Conditioning and cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Hair cleansing conditioning compositions of the present invention can be in the form of conventional liquid thickened shampoo, as well in the form of ready to use foam, delivered either from a pump-foamer or from an aerosol bottle. In the case that an aerosol foam preparation is preferred, propellant gas must be added to the formulation. The suitable propellant gasses are carbondioxide, dimethylether and alkanes such as butane propane or their mixtures.

Compositions of the present invention used after cleansing hair can be in the form of emulsions, solutions, gels and dispersions. In the case that solutions and/or gels forms are preferred the appearance can be either with a transparent or opaque. As a product form, foam is as well suited when packed into a pressurized can or delivered through a pump-foamer (non-aerosol). In the case that an aerosol foam preparation is preferred, propellant gas must be added to the formulation. The suitable propellant gases are carbon dioxide, dimethylether and alkanes such as butane, propane, isobutane or their mixtures.

The emulsion type of conditioners comprise additionally at least one fatty alcohol of the following formula

R₁₄-OH

where R₁₄ is a saturated or unsaturated, branched or non-branched alkyl chain with 8 - 24 C atoms. Concentration of fatty alcohols is usually less than 20%, preferably less than 15%, more preferably in the range of 1 to 10% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

Emulsion type of conditioners comprise in addition to fatty alcohol at least one emulsifier. Suitable emulsifiers are especially non-ionic and cationic surfactants. They can certainly be present also in mixture. Among the non-ionic ones especially preferred are fatty alcohol ethoxylates as mentioned above. Cationic surfactants suitable are mono long chain alkyl carrying ones as disclosed above. Both surfactants are present in the compositions of the present invention in concentration ranges as disclosed above.

Composition comprises organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylene carbonate, ethylene glycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol, ethanol, benzyloxyethanol, Concentration of organic solvents should not exceed 10% by weight, preferably in the range of 0.1 to 7.5% by weight calculated to total composition in compositions designed for cleansing and conditioner / treatment in emulsion form. Higher organic solvent concentrations may be suitable for the preparation such as hair conditioning solution applied by a spraying device. In such case organic solvent concentration can be as high as 50% by weight.

The compositions of present invention can comprise active ingredients such as sequestering agents, natural ingredients, flavonoids, ceramides, pyrrolicdone carboxylic acid or its salts and its esters with an alkyl chain etc.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Natural plant extracts may be incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4^{th} Ed..

Compositions of the present invention optionally may comprise at least one flavone and/or its derivative at a concentration in the range of 0.01% to 2%, by weight, preferably 0.01 to 1.5% and more preferably 0.05 to 1.0% by weight, calculated to total composition.

Suitable non-limiting examples are t-flavanone, quercetin, morin, taxifoline, chatechin, epichatechin and luteolin, The most preferred ones are t-flavanone (trans 3,4'-dimethylflavanonol), quercetin and morin. The flavone and its derivatives are incorporated into the compositions of the present invention either as commercially available pure (the word pure should not be taken as 100% purity and should be understood as flavone derivative enriched raw materials containing other substances) raw material and as well as in the form of natural extracts such as green tea extract, gingko extract, etc.

Composition of the present invention may optionally comprise at least one pyrrolidon carboxylic acid ester of the above formula. The alkyl chain length, in the preferred form, is between 8 and 20 C atoms, more preferably between 12 and 18 C atoms and most preferably between 14 and 18 C atoms.

Some examples to the suitable pyrrolidon carboxylic acid ester are with the alkyl chain of methyl, ethyl, n-propyl, isopropyl, n-butyl, iso butyl, n-hexyl, n-octyl, n-nonyl, n-decyl, ethylhexyl, methylhexyl, capryl, lauryl myristyl, cetyl, octyldodecyl, isostearyl, stearyl, arachidyl, behenyl, oleyl, linoleyl, benzyl, phenoxyethyl. Preferred are n-octyl, n-nonyl, n-decyl, ethylhexyl, methylhexyl, capryl, lauryl myristyl, cetyl, octyldodecyl, stearyl, isostearyl, arachidyl, behenyl, oleyl and linoleyl. More preferred are lauryl myristyl, cetyl, octyldodecyl, stearyl, isostearyl, arachidyl, behenyl, oleyl, and linoleyl. Particularly preferred pyrrolidon carboxylic acid ester is octyldodecyl pyrrolidon carboxylic acid which is available under the trade name Protelan ODPC from Zschimmer & Schwarz.

Concentration of at least one pyrrolidon carboxylic acid ester in the compositions of the present invention is in the range of 0.01 to 5% by weight calculated to total composition. It should be noted that compositions of the present invention can comprise more than one pyrrolidon carboxylic acid ester such as 2 or 3. The above mentioned concentrations refer to the total concentration of the pyrrolidon carboxylic acid esters.

Composition of the present invention comprises pyrrolidon carboxylic acid and/or its salts. In principal any pyrrolidon carboxylic acid salt is suitable within the meaning of the present invention. Suitable examples are aluminium, calcium, copper, magnesium, potassium, sodium and zinc salts of pyrrolidon carboxylic acid. Preferred are aluminium, calcium, magnesium, potassium and sodium salts and more preferred are sodium and potassium salts. The most preferred is sodium salt.

Concentration of pyrrolidon carboxylic acid and/or its salts is in the range of 0.01 to 10% by weight calculated to the total composition. Concentrations mentioned here refer to the total concentration of the pyrrolidon carboxylic acid and/or its salts present in the compositions.

According to the preferred embodiment of the present invention, at least one pyrrolidone carboxylic acid ester of the above formula and pyrrolidone carboxylic acid and/or its salts are comprised in the composition of the present invention at a weight ratio of 1:100 to 1:1, preferably 1:75 to 1:2, more preferably 1:50 to 1:3 and most preferably 1:25 to 1:5.

The pH of the compositions according to the present invention is suitably between 2 and 8.0, preferably in the range of 2.5 to 7.0, more preferably 3 to 6.5 and most preferably 4 to 5.5 measured at ambient temperature with a suitable pH meter. pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Further in preferred embodiment of the present invention, compositions comprise at least one direct dye. Suitable direct dyes are of cationic, anionic and neutral nitro dyes. It should be noted that they can also be used in combination with each other. In other words, a composition according to present invention can comprise an anionic and a cationic dye as well as an anionic and a nitro dye or a cationic and a nitro dye. Certainly the combination of all three dyestuffs is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 4% more preferably 0.05 to 3% and most preferably 0.1 to 2.5% by weight calculated to total composition.

Cleansing compositions of the present invention can also comprise synthetic mica as a further shine enhancer. Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail, the content of the document is included herewith by reference. It also discloses a cleansing composition comprising monoethanolamide surfactant in addition to other surfactants.

Suitable metal oxide or oxides for coating synthetic mica are titanium dioxide, chromium oxide, ferric oxide or mixtures thereof. In the present invention the preferred is synthetic mice coated with titanium dioxide. Such materials are commercially available from Sun Chemical Corporation and known with their INCI names Synthetic Fluorphologopite.

The particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.05 to 7.5%, more preferably 0.1 to 5% and most preferably 0.2 to 2.5% by weight calculated to total composition.

The viscosity of the conditioning shampoo compositions according to the invention is in the range of 500 and about 20,000 mPa.s at 20°C, preferably 1,000 to 10,000, in particular 1,500 to 8,000 mPa.s at 20°C, measured with Höppler viscosimeter.

Viscosity of shampoo compositions can be adjusted with known viscosity enhancers. The preferred ones are monoglycerides such as glyceryl laurate, oleate, PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

Viscosity of the non-cleansing conditioning composition may not be more than 100,000 mPa.s at 20°C measured with Brookfield Rheometer at a shear rate of 10 sec⁻¹.

It should especially be noted that the effects of the inventive compositions become more and more visible after repeated usage.

Therefore a further object of the present invention is a kit comprising at least two compositions, wherein one of the compositions is a cleansing composition based on at least one surfactant and further comprising Polysilicone-15 and Passiflora oil (Passiflora edulis, Passiflora incarnate) and a second composition is non-cleansing conditioning composition comprising Polysilicone-15 and Passiflora oil (Passiflora edulis, Passiflora incarnate).

The following examples are to illustrate the invention, but not to limit. The compositions according to the invention are prepared by mixing the individual components in water, whereby it is also possible to use pre-mixtures of various ingredients.

### Example 1

| | % by weight |
|---|---|
| Polysilicone-15 | 0.5 |
| Passiflora incarnata seed oil | 0.5 |
| Ethanol | 20 |
| Water | to 100 |

The above composition was tested on a coloured hair tress for its protection effect. Therefore, comparative compositions were prepared which comprise either none of the active compounds, or only one of the active compounds at twice higher concentration in order to keep the active matter concentration constant. The compositions were used as leave in compositions, i.e. tresses were dried without rinsing off. The hair tresses obtained were exposed to UV light for XX hours and before and after UV light exposure colour measurements were carried out with a laboratory equipment supplied by Minolta and colour differences (ΔE values) were calculated between the UV exposed tresses and non-exposed tresses using the known equation. Results are presented below.

| Composition comprising | ΔE |
|---|---|
| Ethanol only | 5.95 |
| Polisilicone-15 only | 4.22 |
| Passiflora incarnata seed oil | 4.86 |
| Example 1 as above | 3.03 |

It should be noted for the above results that the lower the number the higher the effect. It is clear from the above results that the most effective composition is the Example 1. The effect observed clearly indicates that there is a synergistic interaction between Polyisliocne-15 and Passiflora incarnata seed oil as the effect observed in combination is not sum of the effects of individual compounds.

### Example 2

| | |
|---|---|
| Sodium lauryl ether sulfate | 11.0 (% by wt.) |
| Coco glucoside | 4.0 |
| Cocoamidopropyl betaine | 1.5 |
| Cationic polymer (Polyquaternium-10) | 0.2 |
| Polysilicone-15 | 0.5 |
| Passiflora incarnata seed oil | 0.5 |
| Perfume, preservative | q.s |
| PEG-60-hydrogenated castor oil | 0.5 |
| PEG-18 Glyceryl cocoate/oleate | 2.0 |
| Citric acid/sodium hydroxide | q.s to pH 5.0 |
| Water | ad100.0 |

The above composition is prepared by combining surfactants and mixing them till homogeneity with part of water. Cationic polymer was dissolved in water with heat and added to surfactant mixture. Polysilicone-15 and PEG-18 Glyceryl cocoate/oleate was added one by one directly to the mixture. Subsequently PEG-60-hydrogenated castor oil mixed with fragrance and Passiflora incarnata seed oil and added tom the mixture. Finally remaining water was added and pH was adjusted to 5.0.

The above composition was tested in a half side blind test against a composition not comprising Passiflora incarnata seed oil and Pofysilicone-15. 10 volunteers having a shoulder length hair participated to the test and a hairdresser carried out washing using equal amount of shampoo composition for both sides. Afterwards the hair conditioning effect of the shampoo was evaluated by hairdresser.

Following results were observed.

**Table I: Results of half side test. The numbers presents the preference. In case of no preference it should be understood that both sides do not differentiate noticeably.**

| | **Inventive** | **Comparative** | **No preference** |
|---|---|---|---|
| **Wet Hair** | | | |
| Combability | 7 | 3 | 0 |
| Smoothness | 7 | 1 | 2 |
| Roughness | 1 | 4 | 5 |
| | | | |

| **Dry Hair** | | | |
|---|---|---|---|
| Combability | 7 | 1 | 2 |
| Smoothness | 8 | 1 | 1 |
| Roughness | 1 | 1 | 8 |
| Managability | 7 | 2 | 1 |
| Volume | 5 | 3 | 2 |
| Body | 7 | 2 | 1 |
| Shine | 8 | 0 | 2 |

From the above results it is clear that the inventive composition improves hair manageability, combability, smoothness, volume, body and shine.

In a further two test series; the shampoo composition comprising only one of the two ingredients was tested against a composition not comprising any of the two ingredients. With the two ingredients Passiflora incarnata seed oil or Polysilicone-15 are meant. The concentration of the compound present in the composition was adjusted to the total concentration of both ingredients. It was observed that the above results could not be obtained with only one of the two ingredients. In both cases it was observed that the inventive composition was conditioning hair in a much better way and this effect is a synergistic effect.

Similar results were observed with the shampoo compositions below.

### Example 3

| | |
|---|---|
| Cetylstearylalcohol | 5.0 (% by weight) |
| Stearyltrimethylammoniumchlorid | 2.0 |
| Benzylalcohol | 2.5 |
| Polysilicone-15 | 0.8 |
| Passiflora incarnata seed oil | 1.0 |
| Fragrance, preservative | q.s. |
| Citric acid | 1.0 |
| Wasser | ad 100.0 |

The pH of the composition is 3.0.

Above composition was tested in a half side test against compositions not comprising Polysilicone-15 and Passiflora incarnate seed oil. The test was carried out with 10 female volunteers having shoulder length hair. Volunteers' hair was washed with a conventional shampoo composition and towel dried and the equal amount of above composition and the comparative without the active ingredients were applied to each side and processed for 5 min. Afterwards the hair was rinsed off and evaluated by a hair dresser for its conditioning properties.

Following results were observed.

**Table II: Results of half side test. The numbers presents the preference. In case of no preference it should be understood that both sides do not differentiate noticeably.**

| | **Inventive** | **Comparative** | **No preference** |
|---|---|---|---|
| **Wet Hair** | | | |
| Combability | 8 | 1 | 1 |
| Smoothness | 8 | 0 | 2 |
| Roughness | 0 | 2 | 8 |
| | | | |

| **Dry Hair** | | | |
|---|---|---|---|
| Combability | 6 | 2 | 2 |
| Smoothness | 8 | 2 | 0 |
| Roughness | 0 | 1 | 9 |
| Managability | 8 | 1 | 1 |
| Volume | 7 | 2 | 1 |
| Body | 8 | 1 | 1 |
| Shine | 7 | 1 | 2 |

From the above results it is clear that the inventive composition improves hair manageability, smoothness, volume, body and shine. Improvement of combability was slightly less pronounced than other properties.

In a further two test series; the conditioner composition comprising only one of the two ingredients was tested against a composition not comprising any of the two ingredients. With the two ingredients Polysilicone-15 and Passiflora incarnate seed oil are meant. The concentration of the compound present in the composition was adjusted to the total concentration of both ingredients. It was observed that the above results could not be obtained with only one of the two ingredients. In both cases it was observed that the inventive composition was conditioning hair in a much better way and this effect is a synergistic effect.

At the same time protection effect against UV exposure was also confirmed with the same method as it was done in Example 1.

Similar results were observed with conditioner compositions below.

### Example 4

### Foam conditioner

| | |
|---|---|
| Quaternium-80 | 0.2 (Gew.-%) |
| Polyquaternium-11 | 0.7 |
| PEG-60-hydrogenated ricinus oil | 0.5 |
| Fragrance, preservative | q.s. |
| Polysilicone-15 | 0.15 |
| Passiflora incarnata seed oil | 0.15 |
| Lactic acid | 0.7 |
| Wasser | ad 100.0 |

pH of the composition is adjusted to 3.4. The composition is suitable for leave-in and rinse off. In leave-in application, amount (whole composition for as a conditioner applied onto hair) used is obviously less than in the case of a rinse of application. The composition is packed into an aerosol can with 90/10 ratio, by weight, liquid composition to propellant. As propellant propane, butane mixture is used.

10 female volunteer were asked to use the above composition at home after washing their hair with their usual shampoo and using the above composition as leave in conditioner by applying on towel dry hair. The comments after a 3 week usage period were
- my hair feels softer,
- I have more hair when I touch my hair,
- My hair moves when I move my head,
- My hair looks shinier
- I can comb my hair very easily

Similar results were obtained with the following examples.

### Example 5

### Conditioner

| | |
|---|---|
| Cetylstearylalcohol | 10.0 (% by weight) |
| Dioleoylethyldimethylammonium ethosulfate | 0.5 |
| Ceteareth 20 | 3.0 |
| Panthenol | 0,4 |
| Phenyltrirmethicone | 0.25 |
| Hydroxypropyl Guar Hydroxypropyltrimonium | |
| Chloride | 0.75 |
| Polysilicone-15 | 0.5 |
| Passiflora incarnata seed oil | 0.15 |
| Basic red 51 | 0.10 |
| Basic red 76 | 0.15 |
| Fragrance, preservative | q.s. |
| Malic acid | 0.8 |
| Wasser | ad 100.0 |

The pH of the composition is 3.4.

The above composition gave in addition to improved manageability, elasiticity and body, excellent red shimmer on medium blonde hair.

## Claims

1. Composition for hair **characterized in that**, it comprises polysilicone-15 and Passiflora oil (Passiflora edulis, Passiflora incarnate).

2. Composition according to claim 1 **characterized in that** it comprises at least one surfactant selected from anionic, nonionic and amphoteric ones.

3. Composition according to claim 2 **characterized in that** it comprises at least one anionic surfactant, at least one nonionic surfactant and at least one amphoteric surfactant.

4. Composition according to claims 1 to 3 **characterized in that** it comprises at least one fatty alcohol according to the formula
R₁₄-OH
wherein R₁₄ is a saturated or unsaturated, branched or non-branched alkyl chain with 8 - 24 C atoms and at least one emulsifier selected from non-ionic and cationic surfactants.

5. Composition according to any of the preceding claims **characterized in that** it comprises at least one conditioning compound selected from oily substances, nonionic substances, cationic amphiphilic ingredients, cationic polymers or their mixture.

6. Composition according to any of the preceding claims **characterized in that** oily substances are selected from silicone oils and synthetic oils.

7. Composition according to any of the preceding claims **characterized in that** it comprises either a cationic polymer as a conditioning compound or a cationic surfactant as a conditioning compound according to following formula 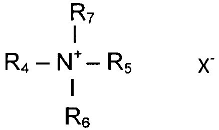 where R₄ is a saturated or unsaturated, branched or straight alkyl chain with 8-22 C atoms or
R₈ CO NH (CH₂)ₙ
where R₈ is saturated or unsaturated, branched or straight alkyl chain with 7-21 C atoms and n has value of 1 - 4,
or
R₉ CO O (CH₂)ₙ
where R₉ is saturated or unsaturated, branched or straight alkyl chain with 7-21 C atoms and n has value of 1 - 4, and
R₅ is hydrogen or unsaturated or saturated, branched or straight alkyl chain with 1 - 22 C atoms or
R₈ CO NH (CH₂)ₙ
or
R₉ CO O (CH₂)ₙ
where R₈, R₉ and n are same as above.
R₆ and R₇ are hydrogen or lower alkyl chain with 1 to 4 carbon atoms, and X is anion such as chloride, bromide, methosulfate, or their mixture.

8. Composition according to any of the preceding claims **characterized in that** it comprises at least one direct dye.

9. Use of composition according to any of the preceding claims for protecting hair from damaging effects of UV light and for improving manageability, elasticity, volume, body and shine of hair.

10. Process for conditioning hair wherein a cleansing composition comprising polysilicone-15 and Passiflora oil (Passiflora edulis, Passiflora incarnate) is applied onto hair and rinsed off from hair and a second composition without cleansing effect comprising polysilicone-15 and Passiflora oil (Passiflora edulis, Passiflora incarnate) is applied onto hair and optionally rinsed off from hair after certain processing time.

11. Kit for conditioning hair **characterized in that** it comprises a first cleansing composition comprising polysilicone-15 and Passiflora oil (Passiflora edulis, Passiflora incarnate) and a second non-cleansing composition comprising polysilicone-15 and Passiflora oil (Passiflora edulis, Passiflora incarnate).

## Patentansprüche

1. Zusammensetzung für Haare, **dadurch gekennzeichnet, dass** sie Polysilikon-15 und Passiflora-Öl (Passiflora edulis, Passiflora incarnata) aufweist.

2. Zusammensetzung nach Anspruch 1, **dadurch gekennzeichnet, dass** sie mindestens ein Tensid aufweist, ausgewählt aus anionischen, nichtionischen und amphoteren.

3. Zusammensetzung nach Anspruch 2, **dadurch gekennzeichnet, dass** sie mindestens ein anionisches Tensid, mindestens ein nichtionisches Tensid und mindestens ein amphoteres Tensid aufweist.

4. Zusammensetzung nach Anspruch 1 bis 3, **dadurch gekennzeichnet, dass** sie mindestens einen Fettalkohol der Formel
R₁₄-OH,
wobei R₁₄ für eine gesättigte oder ungesättigte, verzweigte oder unverzweigte Alkylkette mit 8 bis 24 C-Atomen steht, und mindestens einen Emulgator aufweist, ausgewählt aus nichtionischen und kationischen Tensiden.

5. Zusammensetzung nach einem der vorhergehenden Ansprüche, **dadurch gekennzeichnet, dass** sie mindestens eine Konditionierungsverbindung aufweist, ausgewählt aus öligen Substanzen, nichtionischen Substanzen, kationischen amphiphilen Inhaltsstoffen, kationischen Polymeren oder deren Gemisch.

6. Zusammensetzung nach einem der vorhergehenden Ansprüche, **dadurch gekennzeichnet, dass** ölige Substanzen aus Silikonölen und synthetischen Ölen ausgewählt sind.

7. Zusammensetzung nach einem der vorhergehenden Ansprüche, **dadurch gekennzeichnet, dass** sie entweder ein kationisches Polymer als eine Konditionierungsverbindung oder ein kationisches Tensid als eine Konditionierungsverbindung der folgenden Formel 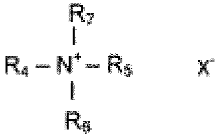 aufweist, wobei R₄ für eine gesättigte oder ungesättigte, verzweigte oder lineare Alkylkette mit 8 bis 22 C-Atomen oder
R₈ CO NH (CH₂)ₙ,
wobei R₈ für eine gesättigte oder ungesättigte, verzweigte oder lineare Alkylkette mit 7 bis 21 C-Atomen steht und n einen Wert von 1 bis 4 aufweist,
oder
R₉ CO O (CH₂)ₙ
steht, wobei R₉ für eine gesättigte oder ungesättigte, verzweigte oder lineare Alkylkette mit 7 bis 21 C-Atomen steht und n einen Wert von 1 bis 4 aufweist, und
R₅ für Wasserstoff oder eine ungesättigte oder gesättigte, verzweigte oder lineare Alkylkette mit 1 bis 22 C-Atomen oder
R₈ CO NH (CH₂)ₙ
oder
R₉ CO O (CH₂)ₙ
steht, wobei R₈, R₉ und n für dasselbe wie oben stehen,
R₆ und R₇ für Wasserstoff oder eine niedere Alkylkette mit 1 bis 4 Kohlenstoffatomen stehen und X für ein Anion wie Chlorid, Bromid, Methosulfat oder deren Gemisch steht.

8. Zusammensetzung nach einem der vorhergehenden Ansprüche, **dadurch gekennzeichnet, dass** sie mindestens einen Direktfarbstoff aufweist.

9. Verwendung einer Zusammensetzung nach einem der vorhergehenden Ansprüche zum Schützen von Haaren vor schädlichen Wirkungen von UV-Licht und zum Verbessern der Handhabbarkeit, der Elastizität, des Volumens, der Fülle und des Glanzes von Haaren.

10. Verfahren zum Konditionieren von Haaren, wobei eine Reinigungszusammensetzung, welche Polysilikon-15 und Passiflora-Öl (Passiflora edulis, Passiflora incarnata) aufweist, auf die Haare aufgebracht wird und aus den Haaren ausgespült wird und eine zweite Zusammensetzung ohne Reinigungswirkung, welche Polysilikon-15 und Passiflora-Öl (Passiflora edulis, Passiflora incarnata) aufweist, auf die Haare aufgebracht und nach einer bestimmten Einwirkungszeit gegebenenfalls aus den Haaren ausgespült wird.

11. Kit zum Konditionieren von Haaren, **dadurch gekennzeichnet, dass** es eine erste Reinigungszusammensetzung aufweist, welche Polysilikon-15 und Passiflora-Öl (Passiflora edulis, Passiflora incarnata) aufweist, und eine zweite nicht reinigende Zusammensetzung aufweist, welche Polysilikon-15 und Passiflora-Öl (Passiflora edulis, Passiflora incarnata) aufweist.

## Revendications

1. Composition pour cheveux **caractérisée en ce qu'**elle comprend du polysilicone-15 et de l'huile de passiflore (Passiflora edulis, Passiflora incarnate).

2. Composition selon la revendication 1, **caractérisée en ce qu'**elle comprend au moins un tensioactif choisi parmi les tensioactifs anioniques, non ioniques et amphotères.

3. Composition selon la revendication 2, **caractérisée en ce qu'**elle comprend au moins un tensioactif anionique, au moins un tensioactif non ionique et au moins un tensioactif amphotère.

4. Composition selon les revendications 1 à 3, **caractérisée en ce qu'**elle comprend au moins un alcool gras selon la formule
R₁₄-OH
où R₁₄ est une chaîne alkyle saturée ou insaturée, ramifiée ou non ramifiée, avec 8 à 24 atomes C, et au moins un émulsifiant choisi parmi des tensioactifs non ioniques et cationiques.

5. Composition selon l'une quelconque des revendications précédentes, **caractérisée en ce qu'**elle comprend au moins un composé de conditionnement choisi parmi des substances huileuses, des substances non ioniques, des ingrédients amphiphiles cationiques, des polymères cationiques ou leur mélange.

6. Composition selon l'une quelconque des revendications précédentes, **caractérisée en ce que** les substances huileuses sont choisies parmi des huiles de silicone et des huiles synthétiques.

7. Composition selon l'une quelconque des revendications précédentes, **caractérisée en ce qu'**elle comprend soit un polymère cationique en tant que composé de conditionnement, soit un tensioactif cationique en tant que composé de conditionnement selon la formule suivante 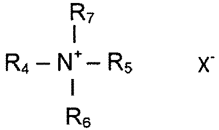 où R₄ est une chaîne alkyle saturée ou insaturée, ramifiée ou linéaire, avec 8 à 22 atomes C, ou un groupe
R₈ CO NH (CH₂)ₙ
où R₈ est une chaîne alkyle saturée ou insaturée, ramifiée ou linéaire avec 7 à 21 atomes C et n a une valeur de 1 à 4,
ou un groupe
R₉ CO O (CH₂)ₙ
où R₉ est une chaîne alkyle saturée ou insaturée, ramifiée ou linéaire, avec 7 à 21 atomes C et n a une valeur de 1 à 4, et
R₅ est un atome d'hydrogène ou une chaîne alkyle insaturée ou saturée, ramifiée ou linéaire, avec 1 à 22 atomes C, ou un groupe
R₈ CO NH (CH₂)ₙ,
ou un groupe
R₉ CO O (CH₂)ₙ
où R₈, R₉ et n sont identiques à ci-dessus.
R₆ et R₇ sont des atomes d'hydrogène ou des chaînes alkyles légères avec 1 à 4 atomes de carbone, et X est un anion tel que le chlorure, le bromure, le méthosulfate ou leur mélange.

8. Composition selon l'une quelconque des revendications précédentes, **caractérisée en ce qu'**elle comprend au moins un colorant direct.

9. Utilisation d'une composition selon l'une quelconque des revendications précédentes pour la protection des cheveux contre des effets d'un endommagement par la lumière UV et pour améliorer la discipline, l'élasticité, le volume, le corps et l'éclat des cheveux.

10. Procédé de conditionnement des cheveux, dans lequel une composition de nettoyage comprenant du polysilicone-15 et de l'huile de passiflore (Passiflora edulis, Passiflora incarnate) est appliquée sur les cheveux et est enlevée des cheveux par rinçage et une seconde composition sans effet nettoyant comprenant du polysilicone-15 et de l'huile de passiflore (Passiflora edulis, Passiflora incarnate) est appliquée sur les cheveux et est éventuellement enlevée des cheveux par rinçage après un certain temps de traitement.

11. Kit de conditionnement des cheveux **caractérisé en ce qu'**il comprend une première composition de nettoyage comprenant du polysilicone-15 et de l'huile de passiflore (Passiflora edulis, Passiflora incarnate) et une seconde composition non nettoyante comprenant du polysilicone-15 et de l'huile de passiflore (Passiflora edulis, Passiflora incarnate).
